# DEMANDE DE BREVET EUROPEEN

(11) **EP 3 852 117 A1**
(43) Date de publication de la demande: **21.07.2021**
(21) Numéro de dépôt: 21151482.3
(22) Date de dépôt: 14.01.2021
(51) Int. Cl.: G16H 20/00

(54) **SYSTÈME MULTI-AGENTS D'OPTIMISATION DES SOINS DE LA PEAU AVEC UNE INTELLIGENCE ARTIFICIELLE DISTRIBUÉE**

(30) Priorité: 14.01.2020 EP 20151742
(71) Demandeur: Daigne, Michel, 84210 Saint Didier (FR); Lasserre, Gilles Henri, 75015 Paris (FR)
(72) Inventeur: Daigne, Michel, 84210 Saint Didier (FR); Lasserre, Gilles Henri, 75015 Paris (FR)
(74) Mandataire: Icosa

(57) **Abrégé**

La présente invention concerne un système de suivi dermatologique à des fins diagnostiques, thérapeutiques et/ou pronostiques d'un sujet, le dit système comprenant au moins une première unité d'enregistrement, de stockage et d'affichage de données personnelles et cliniques, une seconde unité d'enregistrement, de stockage et d'affichage de données personnelles et cliniques, un moyen de mesure ou d'acquisition de paramètres physiques, chimiques, et/ou biologiques de l'environnement dudit sujet, une unité de traitement informatique connectée auxdites première et seconde unités et au moyen de mesure, ladite unité de traitement pouvant comprendre une technologie d'intelligence artificielle distribuée et une technologie de chaine de blocs, et étant apte à alimenter une base de connaissances médicales informatisée sur les soins, les états de santé et les mesures d'hygiène de la peau selon ce qui l'affecte, et à être alimentée en données par ladite base, caractérisé en ce que ladite unité de traitement informatique comprend en outre au moins un programme apte à générer, à partir desdites données, desdits paramètres physiques, chimiques, et/ou biologiques, et desdites connaissances médicales informatisées, une consigne personnelle afin d'influer sur les états de santé et les mesures d'hygiène de la peau dudit sujet selon ce qui l'affecte.

## Description

### DOMAINE DE L'INVENTION

La présente invention concerne notamment un système de suivi dermatologique utilisant une base de données multifactorielle et compartimentée afin d'améliorer la santé de la peau de la personne qui l'utilise avec ses soignants.

La présente invention concerne en particulier un système utilisant des technologies comme par exemple des dispositifs médicaux, des médicaments, des biotechnologies, des programmes informatiques et algorithmes d'intelligence artificielle, des technologies d'hygiène alimentaire et environnementale, afin d'atteindre les objectifs de ses utilisateurs d'amélioration de la santé de la peau, entre autres.

L'invention concerne également un système de gestion et de valorisation d'un patrimoine de données personnelles intimes, privées et publiques, propre au domaine de la santé de la peau que chaque personne intériorise avec sa barrière cutanée et qu'elle extériorise avec son environnement.

### ÉTAT DE LA TECHNIQUE

Afin de suivre voire d'améliorer la santé de la peau d'un sujet, il existe plusieurs approches se déclinant à la fois via des plateformes internet associées et/ou des applications mobiles, celles-ci proposant sur la base des données obtenues, des conseils d'experts, un diagnostic de peau personnalisé et aussi des témoignages concernant par exemple les soins de la peau de la mère et du nourrisson.

Dans ce domaine technologique, on connait par exemple la demande WO2017103195 qui a pour objet des marqueurs biologiques de la peau des enfants, et en particulier celle des nourrissons, dont l'expression est modifiée quand la peau est sèche. De tels marqueurs sont présentés comme avantageux parce qu'ils permettent de suivre la réponse de la peau à la déshydratation. Les inventeurs ont développé des procédés d'évaluation de l'efficacité in vitro de formulations sur la prévention des effets de la déshydratation pour la peau d'enfant, utilisant un modèle de peau spécifiquement capable de reproduire les caractéristiques de la peau des enfants.

Cette approche séquentielle ne permet pas une réactivité à la hauteur des enjeux de la santé de la peau et l'optimisation de ses soins n'en est que plus hors d'atteinte. En outre, il s'agit d'une solution menant à l'ajout, dans une liste déjà longue, de nouvelles prescriptions et conseils de médicaments et produits d'hygiène. Cette solution n'évite donc pas tous les inconvénients tenant aux effets iatrogènes des interactions biologiques, chimiques et physiques multiples entre ces produits prescrits.

En outre, dans le domaine de la santé de la peau, on connait aussi les dispositifs d'observation de la peau par exemple par microscopie et autres systèmes destinés à des mesures individuelles d'exposition par exemple aux ultraviolets. Dans ce registre, est connue la demande US2019311191 concernant un environnement comprenant des systèmes et des méthodes d'analyse de paramètres cutanés à partir d'images ou de vidéos montrant la peau. À l'aide d'une série de filtres d'image différentiels hiérarchiques (HDIF), il devient possible de détecter différentes caractéristiques de la peau telles que les rides, les taches et la rugosité. Le filtre d'image différentielle hiérarchique calcule ainsi deux améliorations à une image montrant une peau à deux niveaux d'amélioration différents, détermine une image différentielle à l'aide de deux améliorations et calcule l'évaluation d'analyse de peau à l'aide de l'image différentielle. Ces évaluations cutanées sont dites d'une précision comparable aux évaluations réelles des dermatologues.

Cette solution vise clairement un perfectionnement esthétique de la peau selon des critères physiques, mentaux et sociaux subjectifs pouvant n'être pas suffisant pour les problèmes dermatologiques de la personne concernée. En outre, pareille solution peut être d'un accès limité pour la majorité de la population souhaitant un service simple et reconnu socialement pour la santé de la peau. Enfin, cette solution présente un intérêt plus limité lorsqu'une économie de moyens mis en œuvre est primordiale.

On comprend ainsi, à la lecture de l'art antérieur de la nécessité d'avoir une solution technologique présentant une réactivité quasi immédiate, en vie réelle de la personne concernée avec son environnement habituel, aussi bien pour le diagnostic que pour la thérapie et le pronostic. Ainsi, la demande de brevet FR3002346 aborde ce sujet à travers un procédé/système de surveillance d'un dispositif médical à disposition d'un patient, ledit dispositif médical comportant un terminal de communication mettant en œuvre des fonctionnalités logicielles pour accompagner le patient dans un traitement thérapeutique prédéterminé, ledit procédé comportant : une étape de réception des données courantes d'exécution des fonctionnalités logicielles provenant du dispositif médical, une étape de test desdites données courantes par comparaison à des données de référence selon au moins une règle de vigilance prédéterminée associée à un risque pour le patient, et une étape d'émission d'un message de vigilance lorsqu'un tel risque est identifié.

Cette approche extrêmement générique met en œuvre un système applicable à tout type de surveillance. Il génère des alertes quand il est aujourd'hui nécessaire d'avoir une application propre au domaine de la santé de la peau, et cela en permettant, au-delà du diagnostic, de faire de la thérapie et un pronostic.

A la lecture de l'état de l'art, il ressort que, dans le domaine de la santé de la peau, les dermatoses inflammatoires chroniques comme la rosacée, la dermatite atopique et les autres types d'eczéma, l'acné, le psoriasis, etc. et leurs mécanismes biologiques ne sont pas encore totalement compris. Il en résulte de nombreux besoins médicaux non-satisfaits, avec une pharmacopée relativement limitée ou non-spécifique aux pathologies concernées comme par exemple l'utilisation d'immunosuppresseurs dans les pathologies cutanées chroniques. Ces pathologies sont pourtant particulièrement influencées par les milieux de vie et les activités de chaque personne. Si elles sont mal soignées, elles peuvent s'aggraver en lien avec le vieillissement de la peau et le développement d'autres pathologies. L'invention vise à traiter ce type de pathologies ou au moins à atténuer leurs effets indésirables tout en pouvant apporter la preuve de l'efficacité de combinaisons complexes et non testées de produits.

### RÉSUMÉ DE L'INVENTION

La présente invention concerne un système de suivi dermatologique à des fins diagnostiques, thérapeutiques et/ou pronostiques, le dit système comprenant au moins :
- une première unité d'enregistrement, de stockage et d'affichage de données personnelles et cliniques de santé et d'hygiène de la peau, propres à un sujet, ladite première unité étant accessible audit sujet pour lesdits enregistrements,
- une seconde unité d'enregistrement, de stockage et d'affichage de données personnelles et cliniques de santé et d'hygiène de la peau, propres audit sujet, ladite seconde unité étant accessible à au moins un praticien médical pour lesdits enregistrements et étant connectée à ladite première unité,
- un moyen de mesure ou d'acquisition de paramètres physiques, chimiques, et/ou biologiques de l'environnement dudit sujet,
- une unité de traitement informatique connectée auxdites première et seconde unités et au moyen de mesure, ladite unité de traitement étant apte à collecter les données personnelles et cliniques des premières et secondes unités et les paramètres physiques, chimiques et/ou biologiques dudit moyen de mesure, ladite unité étant apte à alimenter une base de connaissances médicales informatisée sur la santé et l'hygiène de la peau et à être alimentée en données par ladite base,
- ladite unité de traitement informatique comprend en outre au moins un programme apte à générer, à partir desdites données personnelles et cliniques de santé et d'hygiène de la peau, desdits paramètres physiques, chimiques, et/ou biologiques, et desdites connaissances médicales informatisées :
   - une consigne personnelle afin d'influer sur les états de santé et les mesures d'hygiène de la peau dudit sujet selon ce qui l'affecte; ou
   - des connaissances médicales augmentées sur les soins, les états de santé et les mesures d'hygiène de la peau selon ce qui l'affecte,
caractérisé en ce que les données personnelles et cliniques de santé et d'hygiène de la peau constituent un parcours de santé.

Préférentiellement, la première unité d'enregistrement est un mobile multifonction, un ordinateur, une tablette tactile et/ou un autre appareil tactile tel qu'une montre, borne ou cabine.

De manière préférentielle, la seconde unité d'enregistrement est un mobile multifonction, un ordinateur, une tablette tactile et/ou un autre appareil tactile tel qu'une montre, borne ou cabine.

De manière encore préférentielle, le moyen de mesure ou d'acquisition de paramètres est un ou plusieurs dispositif(s) médical(aux) connecté(s) et comporte des systèmes électroniques programmables. En outre, il peut présenter des capacités d'apprentissage de type algorithme d'intelligence artificielle. Alternativement, il peut est configuré pour acquérir des données des milieux micro-biotiques cutanés propres audit sujet ou encore pour acquérir des données du milieu abiotique cutané propre audit sujet.

Le moyen de mesure ou d'acquisition de paramètres peut aussi est configuré pour acquérir des données d'activités personnelles et domestiques, professionnelles et sociales propres audit sujet. En outre, il peut aussi est configuré pour acquérir des données d'alimentation propre audit sujet.

De manière alternative, le(les) dispositif(s) médical(aux) est(sont) à usage individuel.

De manière préférentielle l'unité de traitement informatique est connectée auxdites première et seconde unités et au moyen de mesure à travers un réseau informatique local partagé, lesdits réseaux informatiques locaux étant connectés à travers un réseau informatique global partagé comprenant un ensemble de ressources informatiques configurables pour le stockage, la transmission et la sécurité des données.

Une alternative selon l'invention consiste en ce que les transactions relatives aux traitements de données dans ladite unité de traitement informatique s'effectuent avec une technologie de type chaine de blocs *(blockchain en anglais)* dont chaque bloc regroupe les transactions propres à un ou plusieurs épisode(s) de soins de la peau.

De manière préférentielle, la base de connaissances médicales informatisée est alimentée par des connaissances acquises par apprentissage du système de suivi dermatologique et des publications scientifiques.

Système de suivi dermatologique selon l'une quelconque des revendications **1 à 13** dans lequel lesdites première et/ou seconde unités et/ou moyen de mesure sont aptes à mettre en œuvre une ou plusieurs applications d'expérimentation clinique d'au moins un médicament et comportant au moins un protocole d'optimisation de posologie,

De manière encore préférentielle, les première et/ou seconde unités et/ou moyen de mesure sont aptes à mettre en œuvre une ou plusieurs applications d'expérimentation clinique d'au moins un médicament, un produit parapharmaceutique, un produit d'hygiène et/ou un produit cosmétique et de leur(s) influence(s) avec des médicaments. En particulier, les première et/ou seconde unités et/ou moyen de mesure et
i. l'une ou plusieurs applications d'expérimentation clinique d'au moins un médicament et comportant au moins un protocole d'optimisation de posologie ou
ii. l'une ou plusieurs applications d'expérimentation clinique d'au moins un produit parapharmaceutique, un produit d'hygiène et/ou un produit cosmétique et de leur influence avec des médicaments,
sont aptes à personnaliser les indications, contre-indications et conseils sur le médicament, le produit parapharmaceutique, le produit d'hygiène et/ou le produit cosmétique, pour augmenter le service médical, de santé et/ou de beauté rendu.Dans un mode alternatif selon l'invention, celle-ci est appliquée au suivi des muqueuses tapissant les cavités du corps qui sont en continuité avec la peau et l'émail des dents, à des fins diagnostiques, thérapeutiques et/ou pronostiques.

### DÉFINITIONS

Dans la présente invention, les termes ci-dessous sont définis de la manière suivante :
- **sujet du système** : Le sujet est la personne dont on parle et que l'on décrit en vie réelle avec ses problèmes de peau, qui les intériorise avec sa barrière cutanée et les extériorise avec son environnement.
- **suivi médical** : Il concerne un ensemble d'opérations consistant à suivre et à contrôler un processus pour parvenir dans les meilleures conditions au résultat recherché. En médecine clinique, le suivi médical est alors un ensemble d'opérations consistant pour un ou plusieurs praticiens médicaux, à suivre et contrôler un processus clinique ayant des fins diagnostiques, thérapeutiques et/ou pronostiques, pour parvenir dans les meilleures conditions au résultat recherché. Ce suivi et ce contrôle d'un processus clinique par des praticiens médicaux varient avec leurs spécialisations qui s'inscrivent dans un modèle anatomo-physiopathologique d'un organisme humain évoluant avec son environnement. Un exemple de champ de la médecine clinique présentant des grandes analogies anatomo-physiopathologiques est celui de la peau et des muqueuses, issues, comme le système nerveux et les organes sensoriels, de la croissance de l'ectoderme.
- **dermatologique** : Dans le cadre de l'invention, ce terme recouvre non seulement une ou plusieurs spécialisations médicales qui vont faire des prescriptions concernant la peau, mais aussi les autres professions tenant un discours parlé ou écrit au sujet de la santé de la peau définie comme état de complet bien-être non seulement physique et mental, mais aussi social par rapport à la beauté, et économique par rapport à la personnalité, en rapport avec ces prescriptions médicales.
- **système d'aide à la décision médicale :** Ce sont « des applications informatiques dont le but est de fournir aux cliniciens en temps et lieux utiles les données et informations décrivant la situation clinique d'un patient ainsi que les connaissances appropriées à cette situation, correctement filtrées et présentées afin d'améliorer la qualité des soins et la santé des patients ». Les systèmes d'aide à la décision médicale peuvent avoir des fins diagnostiques, thérapeutiques ou pronostiques. Les systèmes à base de connaissances visent à simuler le raisonnement des experts engagés dans une démarche décisionnelle. Ils comportent une base de connaissances qui contient les connaissances médicales théoriques et empiriques nécessaires à la démarche diagnostique ou thérapeutique, au sein d'un domaine d'expertise, une base de faits qui représente les caractéristiques du patient pour lequel on cherche à déterminer le diagnostic ou la prise en charge thérapeutique, un moteur d'inférences qui articule les connaissances de la base de connaissances pour raisonner sur la résolution du problème posé dans la base de faits. Les logiciels d'aide à la prescription médicale sont les plus courants et sont réglementés en tant que dispositifs médicaux.
- **épisode de soins :** Épisode associe, en grec, « en » et « voie, chemin » et signifie «action d'intervenir ». Un épisode de soins est une unité d'action, de temps et d'espace au sein duquel un certain nombre de ressources humaines et matérielles sont mises en œuvre pour résoudre un problème médical donné. En particulier, un épisode de soin comprend une observation de santé et d'hygiène de la peau, par exemple un symptôme ou une image ; une consigne personnelle et une évaluation de l'impact ou du résultat de cette consigne personnelle. Un paiement à l'épisode de soins groupant différents professionnels de santé qui interviennent dans des prises en charge chirurgicales, en établissements de santé ou en ville est en cours d'expérimentation. Ce paiement à l'épisode de soins consiste à rémunérer, par une enveloppe forfaitaire globale et selon un périmètre défini, l'intervention des différents professionnels de santé mobilisés en amont, pendant et après l'intervention chirurgicale.
- **santé et hygiène de la peau :** La définition la plus commune de la santé est celle de l'OMS en 1946 : « la santé est un état de complet bien-être physique, mental et social, et ne consiste pas seulement en une absence de maladie ou d'infirmité ». Les inventeurs se réfèrent à cette définition dans le cadre de l'invention en observant que lorsque le suivi médical d'une personne s'effectue en vie réelle et intègre des facteurs environnementaux constituant des biens économiques, il convient d'ajouter un état de bien-être économique aux états de bien-être physique, mental et social. Le bien-être économique fait l'objet de nombreuses recherches pour pallier les insuffisances d'une mesure de la croissance réduite au PIB.
   L'hygiène est l'ensemble des mesures, des procédés et des techniques mis en œuvre pour préserver et pour améliorer la santé. Les mesures d'hygiène peuvent couvrir tout l'environnement de la personne. La santé de la peau d'une personne peut être ainsi décrite comme un ensemble d'états de bien être résultant de soins dermatologiques associés à des mesures d'hygiène couvrant son environnement.
- **personnalité et beauté :** La personnalité et la beauté sont des distinctions économique et sociale extrinsèques complétant les distinctions physique et mentale intrinsèques de la santé de la peau d'une personne.
   La personnalité est ce qui constitue la personne, qui la rend psychiquement, intellectuellement et moralement distincte de toutes les autres. C'est la fonction par laquelle un individu a conscience de son moi, perçoit l'unité de sa vie psychique et son identité dans le temps. C'est le caractère original qui différencie une personne des autres; aspect sous lequel on la considère. C'est l'aptitude à jouir des droits attachés à la personne et définis par la loi. La barrière cutanée et l'environnement d'une personne sont constitutifs de sa personnalité.
   La beauté intègre une valeur esthétique, ce qui est physiquement beau, et des valeurs en rapport avec cette valeur esthétique, ce qui est mentalement, économiquement et socialement beau. Les canons de beauté sont différents d'une époque à l'autre.
- **parcours de santé** : On a modélisé le parcours de santé de la peau d'une personne en vie réelle comme résultant des soins des affections de sa peau et de mesures d'hygiène sur des paramètres de son environnement ayant un impact sur ces affections. Il s'agit par exemple d'une succession d'épisodes de soin. Chaque épisode de soin comprend une observation de santé et d'hygiène de la peau, par exemple un symptôme ou une image ; une consigne personnelle et une évaluation de l'impact ou du résultat de cette consigne personnelle.
- **établissement de santé de la peau** :
   Un établissement de santé de la peau peut se modéliser comme l'ensemble des parcours de santé de la peau d'une communauté de personnes s'intégrant dans un territoire avec des mesures d'hygiène adaptées à ce territoire par exemple en fonction de son climat et de la culture alimentaire qui y prévaut. C'est par exemple les projets de communautés professionnelles territoriales de santé associant des dermatologues, des pharmaciens et des médecins généralistes.
   Une telle modélisation du parcours et de l'établissement de santé de la peau permet de mettre en œuvre une recherche opérationnelle visant à optimiser en vie réelle les soins dermatologiques en simulant des mesures d'hygiène sur des paramètres de l'environnement d'une personne et d'une communauté de personnes dans un territoire.
- **système de connaissances d'un sujet** : Les connaissances sont indissociables du sujet auquel elles se rapportent. Les informations sont à la fois des objectivations des connaissances d'un sujet et des données du sujet contextualisées. Une information dans le cadre de l'invention est une donnée interprétée à travers la connaissance et dans le contexte du sujet
- **données à caractère personnel :** Dans le cadre de l'invention, les données à caractère personnel sont définies comme suit : « toute information se rapportant à une personne identifiée ou pouvant l'être de manière directe ou non, par un ou plusieurs éléments se référant à son identité » (RGPD-Règlement Général sur la Protection des Données dans le cadre juridique de l'Union Européenne).
- **données à caractère personnel de santé** : Il s'agit là de « données relatives à la santé physique ou mentale, passée, présente ou future, d'une personne physique - y compris la prestation de services de soins de santé - qui révèlent des informations sur l'état de santé de cette personne » (RGPD). Elles constituent un noyau de données au sein du réseau des données à caractère personnel.
- **données non personnelles** : Il s'agit là des données qui au départ ne concernaient pas une personne physique identifiée ou identifiable ; ce sont les données qui étaient initialement des données à caractère personnel mais qui ont ensuite été rendues anonymes.
   Une donnée à caractère personnel ne peut être regardée comme rendue anonyme que lorsque l'identification de la personne concernée, directement ou indirectement, devient impossible que ce soit par le responsable du traitement ou par un tiers. Tel n'est pas le cas lorsqu'il demeure possible d'individualiser une personne ou de relier entre elles des données résultant de deux enregistrements qui la concernent.
- **données personnelles et non personnelles :** Si les deux types de données personnelles et non personnelles sont « inextricablement liés » (i.e. la séparation entre les deux serait impossible ou considérée comme économiquement inefficace ou techniquement impossible par le responsable de traitement), il faut respecter le RGPD pour toutes les données de l'ensemble. Il en va de même pour les traitements de données concernant la santé (la frontière étant floue entre les deux types de données traitées, par exemple, dans le cadre d'une application de santé).
- **données cliniques** : Elles sont transversales et en rapport avec les données personnelles de santé, les données personnelles et les données non personnelles. Elles permettent de délimiter le noyau, le système, des données personnelles de santé du réseau des données personnelles dans l'ensemble des données non personnelles (modèle de cellule eucaryote des données personnelles).
- **mobile multifonction :** Le mobile multifonction est ce qui est couramment appelé « *smart phone* ».
- **système multi-agents et intelligence artificielle distribuée :** Ils sont composés d'entités autonomes (les agents) qui interagissent entre elles et avec un environnement dans lequel elles évoluent. Certains de ces agents peuvent être des personnes physiques ou morales, leurs représentants (avatars) ou des machines. S'il y a moins de trois agents, on parle plutôt d'interaction homme/machine, ou machine/machine que de systèmes multi-agents.
- **intelligence artificielle distribuée :** Les domaines complexes et hétérogènes tels que l'aide à la décision dans des situations subtiles, la reconnaissance et la compréhension de formes dans des environnements mouvants, la conduite de processus ont révélé les limites de l'approche classique de l'Intelligence Artificielle (IA) au singulier fondée sur une centralisation de l'expertise au sein d'un seul et unique système expert. Les recherches dans ce domaine ont abouti à la naissance d'une nouvelle discipline : l'Intelligence Artificielle Distribuée (IAD) s'appliquant à un Système Multi-Agents (SMA). Avec une intelligence artificielle distribuée, un ensemble d'entités autonomes, appelées agents, interagissent pour mener à bien une tâche contribuant à la résolution d'un problème complexe. De façon générale, un agent est une entité informatique, située dans un environnement, et qui agit d'une façon autonome pour atteindre les objectifs pour lesquels elle a été conçue.
- **programmes informatiques et algorithmes d'intelligence artificielle :** Le système de suivi dermatologique selon l'invention est un système numérique comportant un ensemble de programmes informatiques et un réseau d'algorithmes concourant à une intelligence artificielle distribuée se rapportant à des équations mathématiques qui seules ont la capacité à représenter le réel, ou du moins un fragment de réalité, en le décrivant et en anticipant, voir en expliquant son comportement.
- **technologie de chaîne de blocs dite *blockchain* en anglais :** C'est une technologie de stockage et de transmission d'informations, transparente, sécurisée, et fonctionnant sans organe central de contrôle. Les transactions effectuées entre les utilisateurs du réseau sont regroupées par blocs. Une fois le bloc validé, il est horodaté et ajouté à la chaîne de blocs. La transaction est alors visible pour le récepteur ainsi que l'ensemble du réseau. Elle peut servir pour le transfert d'actifs, en tant que registre de produits et d'actifs, pour des contrats intelligents ou smart contracts en anglais.
- **milieux micro-biotiques et milieu abiotique individuels :** Ce sont les milieux constituant l'écosystème individuel au sein duquel se réalisent les phénomènes d'auto organisation anatomo physiopathologiques d'un être humain, par exemple les phénomènes immunologiques, d'oxygénation ou de rythme circadien.
   La biocénose de cet écosystème individuel est le réseau de ses milieux microbiotiques. Les milieux microbiotiques, constitués de micro-organismes de type bactéries, microchampignons, protistes, virus, etc... sont propres à un organe comme par exemple l'appareil respiratoire et l'appareil digestif, la peau et les muqueuses, l'appareil urogénital, etc... Ils interagissent avec l'organisme qui les héberge, avec des fonctions de fermentation, digestion de fibres, synthèse de vitamines, barrière contre divers pathogènes ainsi qu'une implication dans le système immunitaire.
   Le biotope de cet écosystème individuel est l'ensemble de son milieu abiotique. Ce milieu abiotique est constitué de rayonnements électromagnétiques (lumière, etc.), d'ondes mécaniques (sons, etc.) et des composants de l'air ainsi que de leurs pollutions respectives.
- **activités d'une personne et son environnement :** Les activités d'une personne sont par définition personnelles et peuvent être domestiques, professionnelles et/ou sociales. Cette différenciation des activités est en accord avec les règlementations s'appliquant au traitement de données à caractère personnel, automatisé en tout ou en partie, ainsi qu'au traitement non automatisé de données à caractère personnel contenues ou appelées à figurer dans un fichier.
   En effet, ces règlementations ne s'appliquent pas au traitement de données à caractère personnel effectué par une personne physique dans le cadre d'une activité strictement personnelle ou domestique.
   L'environnement d'une personne est alors la limite, l'enveloppe de ces activités personnelles et domestiques, professionnelles et sociales. Adapter son environnement revient pour une personne à adapter ses activités.
- **conduite alimentaire d'une personne :** La conduite alimentaire d'une personne selon l'invention s'entend comme une succession de prises alimentaires journalières. Ces prises alimentaires résultent de préparations. Ces dernières peuvent être ménagères, artisanales ou industrielles.
   La qualité nutritionnelle de ces préparations alimentaires est reliée, d'une part, à la présence d'éléments nutritifs essentiels comme les acides aminés essentiels, acides gras insaturés, fibres alimentaires, micronutriments tels que les vitamines, les antioxydants, les minéraux, les substances bioactives et, d'autre part, à leur biodisponibilité.
   Les produits alimentaires servant à ces préparations peuvent être classés selon leur degré de transformation, par exemple selon la classification NOVA distinguant les produits frais ou peu transformés (« *minimally processed»* en anglais), les ingrédients culinaires transformés (« *food ingredient* » en anglais), les produits transformés (« *processedfood* » en anglais), les produits hautement transformés (« *ultraprocessed food »* en anglais).

### BRÈVE DESCRIPTION DE FIGURES

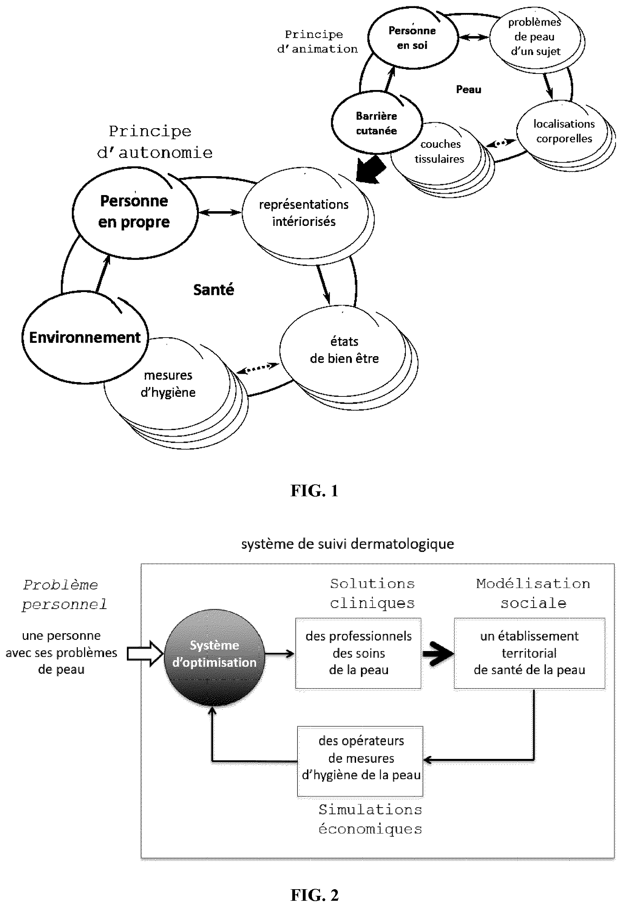

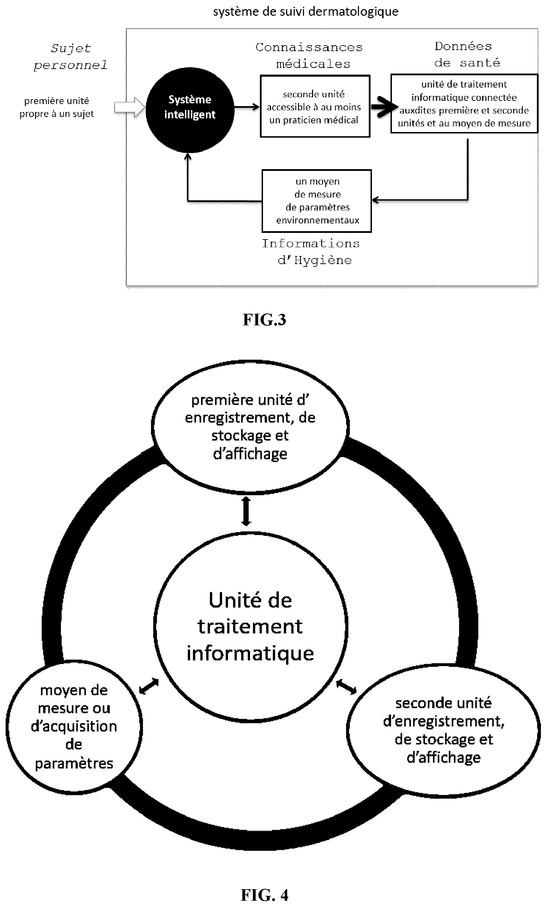

Les figures 1 à 6 schématisent sous forme de cycles le système multi-agents d'optimisation des soins de la peau avec une intelligence artificielle distribuée et une technologie de chaines de blocs selon l'invention.

### DESCRIPTION DÉTAILLÉE ET EXEMPLES

Il est à noter que les figures **1 à 6** de l'invention représentent plusieurs vues illustrant les relations entre les agents concourants au fonctionnement du système multi-agents.

La **figure 1** illustre le parallèle entre le principe d'animation et celui d'autonomie.

L'ensemble des bases de données de l'unité de traitement informatique comme ensemble intégrateur des **figures 2** **et** **3** et central de la **figure 4** est obtenu grâce au mobile multifonction, à l'ordinateur, à la tablette digitale ou autre appareil digital sur lequel la personne a installé sa première unité d'enregistrement, de stockage et d'affichage de données personnelles et cliniques de santé de la peau.

Il est aussi obtenu grâce aux postes de travail sur lesquels le dermatologue, le pharmacien, le médecin généraliste travaillant avec un ou plusieurs autres professionnel des soins de la peau, a installé sa seconde unité d'enregistrement, de stockage et d'affichage de données personnelles et cliniques de santé de la peau avec son système d'aide à la décision dermatologique spécifique, aux systèmes informatiques des opérateurs de chaque catégorie de dispositifs médicaux à usage individuel permettant de prendre des mesures d'hygiène pour la santé de la peau, voire des moyens de mesure ou d'acquisition de paramètres physiques, chimiques, et/ou biologiques tels que des informations météorologiques, des géolocalisations, etc..

Il est aussi obtenu grâce à une unité de traitement informatique des données comportant une unité locale de traitement informatique des données personnelles et cliniques nominatives concourant à un établissement de santé de la peau constitué par une communauté de personnes et de soignants au sein d'un environnement de mesures d'hygiène définies à l'échelle d'un territoire, une unité globale de traitement informatique des données comportant :
▪ un réseau de transmission informatique avec des chaines de blocs *(blockchains* en anglais) dont chaque bloc regroupe les transactions s'appliquant aux données pseudonymisées d'un ou plusieurs épisode(s) de soins de la peau d'une personne ou d'une communauté de personnes réalisé(s) au sein d'un établissement territorial, chaque chaine de blocs ayant une finalité bien définie, par exemple transferts d'actifs, registres d'actifs et produits, contrats intelligents *(smart contracts* en anglais) pour un paiement à l'épisode de soins, des essais cliniques en vie réelle, une industrialisation de mesures d'hygiène, etc.,
▪ un ensemble de stockage informatique de données anonymisées et non personnelles dans une informatique en nuage *(cloud computing* en anglais),
▪ des systèmes de sécurité informatique assurant les fonctions d'authentification des utilisateurs, de chiffrement de leurs transactions et de protection de leurs données au sein de l'unité de traitement informatique locale et globale.

Cette unité de traitement informatique comprend en outre un programme. Ce programme s'appuie sur les données personnelles et cliniques de santé et d'hygiène de la peau ; sur les paramètres physiques, chimiques, et/ou biologiques ; et sur les connaissances médicales informatisées pour générer :
- soit une consigne personnelle. Cette consigne vise à influer sur les états de santé et les mesures d'hygiène de la peau pour un sujet particulier ;
- soit une connaissance médicale nouvelle contribuant à augmenter les connaissances médicales sur les soins, les états de santé et les mesures d'hygiène de la peau.

Dans l'invention, les données personnelles et cliniques de santé et d'hygiène de la peau constituent un parcours de santé.

Ce parcours de santé comprend par exemple une succession d'épisodes, dans laquelle chaque épisode comprend au moins trois éléments :
- Une observation. Cette observation peut être d'ordre très variée. Par exemple, l'observation est un symptôme observé par un praticien, un résultat d'analyse, une image ou une évaluation par le sujet d'un niveau subjectif (démangeaison, rougeur, sensibilité...).
- Une consigne personnelle. Il s'agit ici d'une consigne générée par l'unité de traitement informatique. Cette consigne peut être d'ordre très variée. Par exemple, la consigne est un traitement médical (principe actif, posologie...), un soin cosmétique, une consigne alimentaire, une consigne de comportement compte tenu des conditions environnementales.
- Une évaluation de l'impact ou du résultat de cette consigne personnelle. Cette évaluation est recueillie a posteriori, après que la consigne personnelle a été appliquée. Cette évaluation peut être d'ordre très variée. Par exemple, l'évaluation est une appréciation de la disparition ou de la diminution des symptômes, une évaluation par le sujet de l'évolution d'un niveau subjectif (démangeaison, rougeur, sensibilité...).

Ainsi, la succession d'épisodes permet pour le sujet de recevoir des consignes personnelles issues des connaissances médicales informatisées et dont la performance est évaluée par rapport à la référence que constituent ces connaissances médicales informatisées. En particulier, si le sujet répond positivement à des consignes personnelles qui avaient été identifiées pour d'autres patients ayant des observations similaires, l'unité de traitement informatique pourra générer une consigne personnelle.

Par ailleurs, l'agrégation des épisodes par l'unité de traitement informatique permet de dégager des tendances générales sur l'efficacité des consignes personnelles et d'optimiser les consignes à générer.

Un parcours de santé est typiquement construit de la manière suivante.

Dans un premier épisode d'amorçage, le sujet utilise la première unité d'enregistrement. Il s'agit d'un mobile multifonction, d'un ordinateur ou d'un appareil tactile tel qu'une tablette, une montre ou une borne installée dans un centre de santé. La première unité d'enregistrement peut aussi comporter une capture d'image, par exemple dans une cabine comparable à une cabine de prise de photographies d'identité.

Un questionnaire en deux parties est alors complété par le sujet. La première partie concerne ses données personnelles et d'identification par le système. La seconde partie concerne ses données cliniques de santé et d'hygiène de la peau. Un élément de localisation (par déclaration de lieu de résidence ou par géolocalisation) est aussi complété, permettant de connaître ensuite les conditions environnementales (ensoleillement, température, taux d'humidité, concentrations en polluants...) du lieu de vie du sujet.

Eventuellement, un praticien utilise une seconde unité d'enregistrement pour compléter les données transmises par le sujet. En particulier, le praticien apporte une quantification sur les données cliniques de santé et d'hygiène de la peau et identifie des symptômes.

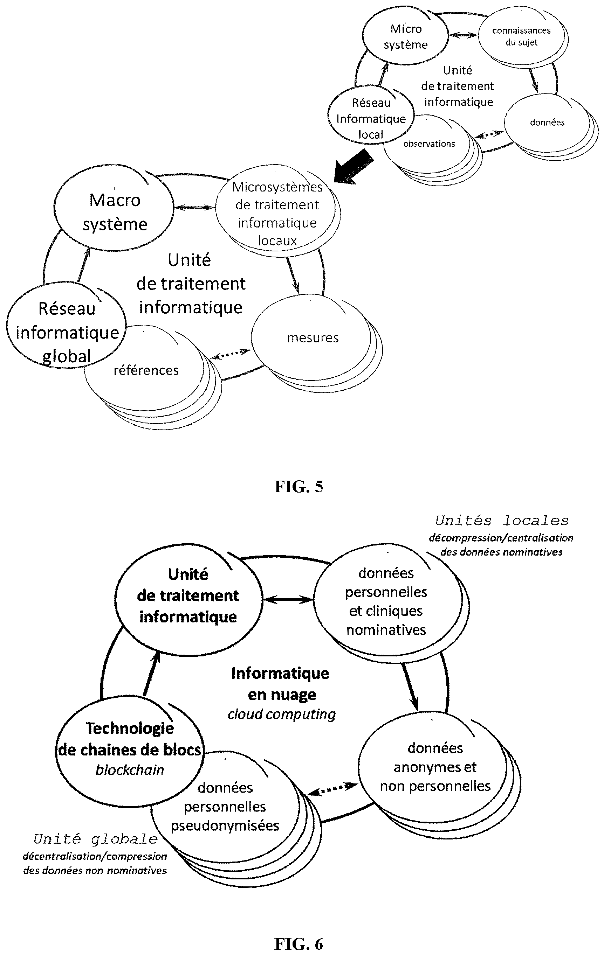

L'ensemble de ces données constitue les éléments de «connaissance du sujet », « données » et « observation » de la **figure 5****.**

L'unité de traitement informatique génère ensuite une consigne personnelle sur la base des connaissances médicales informatisées et des données et des données du sujet.

L'évaluation de l'efficacité de la consigne personnelle est faite plus tard, et vient compléter l'épisode. Cette évaluation est assurée par le sujet et/ou le praticien à l'aide de leur unité d'enregistrement.

Les épisodes suivants reprennent uniquement la seconde partie du questionnaire, la génération d'une consigne personnelle et l'évaluation de l'efficacité de la consigne.

Cette succession d'épisodes regroupés dans un réseau informatique local permet de décrire le système de suivi dermatologique à l'échelle d'un patient, c'est-à-dire d'un « micro système » tels que visible sur la **figure 5****.**

A l'aide de la seconde unité d'enregistrement, le praticien peut à la fois visualiser et intervenir sur la prise en charge d'un patient et aussi comparer des prises en charges différentes. L'ensemble des parcours de soins constitue un établissement de santé.

Par la prise en compte longitudinale, sur un temps long, des épisodes successifs, le parcours de santé permet une meilleure prise en charge du patient, un enrichissement des connaissances médicales et une amélioration de l'établissement de santé.

La **figure 5** représente le système de suivi dermatologique à l'échelle d'un établissement de santé de la peau « macro système », dans lequel l'unité de traitement informatique agrège les données issues des « micro systèmes ». Chaque épisode de soin constitue alors une mesure et un ensemble de références peut être construit, contenant aussi bien les références de soins que les références de symptômes traités par exemple.

En outre, il est possible d'associer aux consignes personnelles un coût (pour le sujet ou pour la société) ce qui permet de mesurer le service rendu médico-économique d'une consigne ou d'une suite de consignes.

La **figure 6** schématise cette unité de traitement informatique locale et globale dont les ressources informatiques peuvent être classiques et/ou quantiques. L'architecture centralisée/déconcentrée localement et décentralisée/concentrée globalement de cette unité de traitement informatique permet d'appliquer des règles de collecte et traitement des données de santé et d'hygiène de la peau distinctes : les règles s'appliquant aux données personnelles et cliniques nominatives dans l'unité de traitement informatique locale propre à un établissement de la santé de la peau à l'échelle d'un territoire, les règles s'appliquant aux données personnelles pseudonymisées à partir des données nominatives des épisodes de soins lors des transactions effectuées avec des chaines de blocs ayant des finalités définies, les règles s'appliquant aux données anonymisées et non personnelles dans une informatique en nuage.

Le système selon l'invention, s'auto alimentant en continu de données personnelles et cliniques de santé de la peau, de connaissances dermatologiques et d'informations d'hygiène, peut suivre les effets des prescriptions médico-chirurgicales et de mesures d'hygiène, et apprendre des différents sujets suivis à l'échelle d'un territoire (unité de traitement informatique locale), à l'échelle d'entreprises industrielles (chaines de blocs) et à l'échelle d'un Etat (informatique en nuage souveraine).

Et la distinction des règles de collecte et traitement des données de santé et d'hygiène de la peau au sein de l'unité locale de traitement informatique locale et de l'unité globale de traitement informatique s'accorde avec des systèmes de sécurité informatique classiques et/ou quantiques pour l'authentification des utilisateurs, le chiffrement de leurs transactions et la protection de leurs données. Par exemple un système de cryptographie quantique à l'échelle locale et de cryptographie post quantique et classique à l'échelle globale.

La présente invention se comprendra mieux à la lecture des deux exemples suivants qui illustrent non-limitativement l'invention.

Un premier exemple d'application du système selon l'invention concerne le suivi de la rosacée. Cette maladie inflammatoire chronique de la peau provoque des rougeurs et des reliefs sur le visage. La rosacée comporte des aspects vasculaires et fibrosants. Ses causes multifactorielles vont de facteurs génétiques, comme une peau claire, à des dysbioses cutanées, comme par exemple des acariens activant une réaction immunitaire anormale, et à des facteurs environnementaux comme le froid, la chaleur, ou l'exposition solaire. Son évolution dépend d'interactions entre la peau et les multiples produits de santé, d'hygiène, voire de beauté selon des critères prédéfinis, utilisés tout au long de la vie, ainsi que de déclencheurs alimentaires comme par exemple les aliments riches en histamine.

L'enjeu est donc bien d'optimiser les soins dermatologiques de la rosacée associés à des mesures d'hygiène centrées sur les causes externes précédentes de manière à obtenir les meilleurs résultats possibles en termes de santé de la peau au cours d'épisodes successifs de soins. Le suivi peut en outre permettre d'améliorer l'esthétique de la peau du sujet, en prenant en compte la dimension subjective de cette dernière caractéristique et les critères socio-culturels de beauté dans lesquels se reconnaît le sujet.

La rosacée n'est pas mortelle, mais elle peut être très handicapante pour la vie tant personnelle que professionnelle et sociale.

Les composantes de l'invention ont alors les caractéristiques suivantes :
Lors d'un épisode d'amorçage des soins, la première unité d'enregistrement de stockage et d'affichage de données personnelles et cliniques de santé de la peau selon l'invention permet ainsi de saisir les symptômes d'une rosacée d'un sujet : rougeurs sur le visage (érythème), peau sèche et sensible, avec sensation de brûlure, tendance à rougir facilement sur les joues, mais aussi parfois sur le nez, le front et le menton, bouffées vasomotrices, ou accès de rougeur déclenchées par les émotions et les changements de température et survenant souvent après les repas, petits vaisseaux sanguins apparents sur le nez et les joues (télangiectasies ou couperose), petits boutons rouges et solides (papules) ou remplis de pus (pustules) sur le nez, les joues, le front et le menton, yeux secs, rouges et irrités, nez rouge, enflé et recouvert de nodules, au stade avancé de la maladie.

De plus, un élément de localisation permettant de connaître les conditions environnementales du sujet est ajouté dans la première unité d'enregistrement de stockage et d'affichage de données personnelles et cliniques de santé de la peau.

Cette première unité permet aussi de se connecter avec une unité de traitement informatique apte à collecter les données personnelles et cliniques de ce sujet.

Une seconde unité d'enregistrement, de stockage et d'affichage de données personnelles et cliniques de santé de la peau du sujet et accessible au(x) praticien(s) médical(aux) en rapport clinique avec ce sujet, permet aussi de se connecter avec cette première unité et cette unité de traitement informatique. La première et la seconde unité d'enregistrement, de stockage et d'affichage sont dans cet exemple des mobiles multifonction, ordinateurs, tablettes tactiles ou autres appareils tactiles. Les deux unités susnommées ainsi que l'unité de traitement sont bien entendues connectées via un réseau informatisé.

Cette seconde unité d'enregistrement, de stockage et d'affichage de données personnelles et cliniques de santé de la peau comporte un système d'aide à la décision dermatologique qui permet de faire un diagnostic de rosacée entre ces formes érythémato-télangiectasique, papulo-pustuleuses et hypertrophique (phyma), de faire un pronostic sur son évolution et ses complications par exemple oculaires, de prescrire les traitements médicaux et chirurgicaux en conséquence à travers des propositions de médicaments ou de solutions de type lasers médicaux, etc. et enfin de prescrire des mesures d'hygiène individualisées. Ces mesures d'hygiène vont concerner par exemple des types d'acariens, des facteurs environnementaux comme le froid ou la chaleur, l'exposition solaire et certaines pollutions de l'air, ainsi que des déclencheurs alimentaires.

Sur la base de toutes les données personnelles et cliniques ainsi rassemblées et disponibles sur la rosacée et la santé de la peau du sujet et des connaissances médicales qui leurs sont associées par le système d'aide à la décision médicale, le tout étant accessible dans l'unité de traitement, les dispositifs connectés permettant au sujet et au(x) praticien(s) médical(aux) de prendre des mesures d'hygiène sont personnalisés en conséquence par leurs opérateurs respectifs. Ces dispositifs médicaux connectés et à usage individuel ont pour objet l'auto-traitement d'une aire microbiotique, l'auto-surveillance d'un milieu abiotique, l'auto-régulation d'une conduite alimentaire, l'auto-adaptation d'un champ d'activités.

Cette première unité de traitement d'enregistrement, de stockage et d'affichage de données personnelles et cliniques de santé de la peau d'un sujet et les dispositifs médicaux permettant de prendre des mesures d'hygiène permettent ensuite au sujet d'orienter l'évolution de sa rosacée en suivant les prescriptions de traitements médicaux et chirurgicaux et de mesures d'hygiène de son(ses) praticien(s) médical(aux) effectuées avec la seconde unité d'enregistrement, de stockage et d'affichage de données et son système d'aide à la décision dermatologique.

Le deuxième épisode de soins de la peau et les épisodes suivants au cours desquels est mis en œuvre le système de suivi dermatologique, sont déclenchés par la comparaison entre l'évolution des données personnelles et cliniques sur la rosacée et la santé de la peau du sujet au cours de l'épisode de soins en cours et les prescriptions médico-chirurgicales et de mesures d'hygiène effectuées au début et mis en œuvre au cours de cet épisode de soins.

L'optimisation des soins de la peau est obtenue de trois manières qui se cumulent et se complètent :
Premièrement : par une personne avec ses soignants.

La succession des épisodes de soins lui permet de minimiser sa rosacée et de maximiser la santé de sa peau tout au long de sa vie, en sachant que la rosacée débute après l'âge de l'acné, vers 25 à 30 ans et que le pic de sa fréquence se situe après 45 ans. Cette maximisation de la santé de la peau à partir d'un problème de rosacée sera évaluée à travers la succession des épisodes de soins du sujet, en termes de bien-être physique et mental, mais aussi de personnalité (bien-être économique) et beauté (bien-être social).

Deuxièmement : dans une communauté de personnes et de soignants à l'échelle d'un territoire.

L'intégration de données personnelles et cliniques en associant avec des connaissances médicales et des informations d'hygiène sur la rosacée et la santé de la peau, permet d'acquérir des connaissances médicales nouvelles sur les interactions biologiques, chimiques et physiques et les effets iatrogènes multiples des très nombreux produits utilisés en rapport avec la peau :
- les médicaments traitant les problèmes de vascularisation, de fibrose, de déshydratation et d'infection de la peau : antibactériens, antiparasitaires et anti-inflammatoires sous forme de gels, de crèmes ou de lotions, antibiotiques, etc.
- les produits nettoyant du visage pour les peaux sèches, grasses ou mixtes, et sous forme de gels, mousses, crèmes, savons, etc.
- les produits de maquillage selon le type de peau : fonds de teint, caches cernes, poudre translucide, blush, poudre bronzante, etc.

Les connaissances médicales sur ces interactions vont s'étendre aussi aux polluants et nutriments selon la nature des activités (industries, etc.) et la culture alimentaire dans le territoire de la communauté de personnes et de soignants.

Ces nouvelles connaissances médicales vont nourrir les systèmes d'aide à la décision dermatologique spécifiques aux dermatologues, aux pharmaciens et aux médecins généralistes qui coopèrent lors d'un épisode de soins de la peau. Chacun de ces trois systèmes d'aide à la décision dermatologique comporte un moteur d'inférences diagnostiques, pronostiques et thérapeutiques sur une base de connaissances propre à chacun des domaines d'expertise dans le champ de la rosacée et de la santé de la peau. La base de faits de ce système d'aide à la décision est constituée des données personnelles et cliniques rassemblées et disponibles dans l'unité de traitement informatique. Les bases de connaissances sont constituées d'une part du socle des connaissances acquises de la science et des connaissances avérées rassemblées sous l'autorité des conseils nationaux professionnels des trois spécialités concernées et d'autre part des connaissances élaborées par le système multi-agents d'optimisation des soins de la peau avec des méthodes d'apprentissage automatique (« deep learning ») modélisant grâce à des architectures articulées de différentes transformations non linéaires, les données personnelles et cliniques, les connaissances médicales et les informations d'hygiène rassemblées et disponibles dans l'unité de traitement informatique.

Ces systèmes d'aide à la décision utilisés par le dermatologue, le pharmacien et le médecin généraliste concourant au suivi dermatologique, permettent par exemple de prodiguer des soins du visage avec des produits de santé, d'hygiène et cosmétiques en se basant sur l'expérimentation de différentes combinaisons de ces produits avec des méthodes scientifiques et sur les connaissances acquises sur l'usage de ces produits en vie réelle par le système multi-agent au cours d'épisodes de soins successifs avec des méthodes d'apprentissage automatique.

Troisièmement : avec les dispositifs médicaux connectés permettant de prendre des mesures d'hygiène individuelles et collectives

Le dispositif d'auto-traitement de l'aire du microbiote cutané du visage correspondant à la rosacée : l'immunité innée est la première ligne de défense vis-à-vis des agents infectieux et pathogènes. Elle met en jeu des modules de défense constitutifs comme la barrière peau-muqueuse, et des modules de défense induits comme la phagocytose et la réponse inflammatoire, qui nécessite les cellules phagocytaires et les cytokines. La rosacée est associée à la présence anormalement élevée d'un acarien, le *demodex folliculorum,* lui-même porteur d'une centaine de bactéries différentes qui vont activer des réactions immunologiques.

Le dispositif d'auto-surveillance du milieu abiotique du visage : il sera axée sur l'exposition du visage à la lumière solaire et aux alternances de grands froids et de périodes de grande chaleur, mais aussi à certaines pollutions de l'air. Ces paramètres sont facilement accessibles dans l'unité de traitement informatique par un simple accès internet et une géolocalisation de la personne.

Le dispositif d'auto-adaptation du champ d'activités : il servira plus particulièrement à limiter le stress et la fatigue, à adapter les activités sportives, etc., mais aussi à adapter les activités pour limiter les expositions microbiotiques et abiotiques du visage.

Le dispositif d'auto-régulation de la conduite alimentaire : il servira à contrôler la prise de déclencheurs alimentaires comme les aliments riches en histamine (tomates, agrumes, noix, crustacés, etc.), l'alcool, les épices, les boissons chaudes, le chocolat et des aliments fermentés (choucroute, certains fromages, etc.). L'identification de ces déclencheurs alimentaires peut être faite grâce au système selon l'invention en recoupant des points communs de sujets présentant les mêmes symptômes ou tendances suite aux mêmes prises d'aliments.

Chacun de ces dispositifs médicaux connectés et l'environnement qu'il constituent pour chaque personne et la communauté de personnes au sein de laquelle elle s'identifie, va permettre de faire des simulations de plus en plus précises sur les mesures d'hygiène concourant à la santé de la peau, avec un apprentissage tout au long des épisodes de soins de la peau successifs d'un sujet et un apprentissage entre sujets suivis dans un communauté territoriale d'établissement de santé de la peau. Les soins de la peau prodigués à l'échelle individuelle et collective en seront d'autant optimisés.

Un deuxième exemple d'application du système selon l'invention concerne le suivi de la dermatite atopique. Cette maladie inflammatoire chronique de la peau provoque des rougeurs du visage. La dermatite atopique, aussi appelée eczéma atopique, est une maladie chronique inflammatoire de la peau. Elle se développe préférentiellement chez le nourrisson et l'enfant, mais peut persister voire apparaître parfois chez l'adolescent et l'adulte. Elle est caractérisée par une sècheresse cutanée associée à des lésions de type eczéma (rougeurs et démangeaisons, vésicules, suintement et croûtes) qui évoluent par poussées.

Une prédisposition génétique est vraisemblablement à l'origine d'une altération de la barrière cutanée des patients, causant sécheresse et sensibilité accrue aux agressions. Cette altération de la structure de la peau va en outre permettre aux allergènes de l'environnement (pollens, poussières, savon, etc.) de pénétrer dans l'épiderme et de stimuler le système immunitaire. Ce dernier va réagir de façon excessive à ce qu'il considère comme une agression, déclenchant l'eczéma : rougeurs, démangeaisons, inflammations et suintements.

Ces poussées inflammatoires sont favorisées par des facteurs environnementaux variés : des produits irritants comme le savon, les tissus rêches, certains aliments, un air trop sec, la chaleur, la sueur... Comme pour d'autres pathologies inflammatoires chroniques, le stress a un effet bien réel chez certains patients. Les conflits psychoaffectifs peuvent ainsi entraîner des recrudescences de la dermatite atopique, par exemple à l'adolescence.

L'enjeu est donc bien d'optimiser les soins dermatologiques de la dermatite atopique associés à des mesures d'hygiène centrées sur les causes externes précédentes de manière à obtenir les meilleurs résultats possibles en termes de santé de la peau lors d'épisodes de soins successifs pouvant s'amorcer chez le nourrisson et s'étendre jusqu'à l'âge adulte.

Les composantes de l'invention ont alors les caractéristiques suivantes :
Lors d'un épisode d'amorçage des soins, la première unité d'enregistrement de stockage et d'affichage de données personnelles et cliniques de santé de la peau selon l'invention permet ainsi de saisir les symptômes d'une dermatite atopique d'un sujet : la peau sèche (xérose), source d'inconfort et de démangeaisons (prurit) ; des plaques rouges qui apparaissent par poussées, démangeant encore davantage et s'aggravant si on ne résiste pas à l'envie de les gratter... Chez le bébé, les lésions sont le plus souvent présentes sur les zones rebondies du visage et des membres, sur le cuir chevelu et les fesses. Plus tard, on les retrouve au niveau des plis (cou, derrière les genoux, plis des coudes), des mains et autour de la bouche.

De plus, un élément de localisation permettant de connaître les conditions environnementales du sujet est ajouté dans la première unité d'enregistrement de stockage et d'affichage de données personnelles et cliniques de santé de la peau.

La seconde unité d'enregistrement, de stockage et d'affichage de données personnelles et cliniques de santé de la peau comporte un système d'aide à la décision dermatologique permettant d'accompagner :
- un diagnostic de dermatite atopique, l'évaluation de ses altérations de la barrière cutanée et l'identification de ses facteurs déclenchants,
- un pronostic sur son évolution et ses complications par exemple les colonisations des lésions cutanées par le staphylocoque doré ou le virus de l'herpès, des retards de croissance et des complications ophtalmologiques,
- de prescrire un traitement de fond visant à lutter contre la sécheresse cutanée et à restaurer la fonction de barrière de la peau, par exemple par l'application quotidienne d'émollients (crèmes hydratantes) et un traitement lors d'une poussée visant à réduire l'inflammation et les démangeaisons, pour soulager le patient et éviter une surinfection, et enfin de prescrire des mesures d'hygiène individualisées. Ces mesures d'hygiène vont concerner par exemple concerner des excès d'hygiène et perte de stimulation du système immunitaire à une phase précoce du développement, un lavage excessif de la peau, un habitat très isolé et mal ventilé favorable aux acariens, une présence accrue d'animaux domestiques, des enfants plus souvent gardés en communauté, une évolution et diversification précoce des habitudes alimentaires, une exposition au tabac et aux pollutions urbaines industrielles, etc.

Le deuxième épisode de soins et les épisodes suivants au cours desquels est mis en œuvre le système de suivi dermatologique, sont déclenchés par la comparaison entre l'évolution des données personnelles et cliniques sur la dermatite atopique et la santé de la peau du sujet au cours de l'épisode de soins en cours et les prescriptions de traitements et de mesures d'hygiène effectuées au début de l'épisode de soins en cours.

Les dispositifs médicaux connectés permettant de prendre des mesures d'hygiène individuelles et collectives seront adaptés au suivi de la dermatite atopique :
Le dispositif d'auto-traitement des aires microbiotiques de la peau correspondant à la localisation de la dermatite atopique qui évolue avec l'âge : elle atteint habituellement les joues, le front et le pouce sucé chez le nourrisson, puis les plis des coudes et des genoux chez l'enfant. Parfois, elle persiste ou apparaît chez l'adolescent ou l'adulte, et elle atteint alors fréquemment le visage et le cou.

Le dispositif d'auto-surveillance du milieu abiotique de la peau : il sera axée sur les pollutions de l'air intérieur et extérieur comme des allergènes, des acariens, la présence d'animaux, des pollutions industrielles, etc. mais aussi sur les vêtements comme des tissus trop rêches, etc.

Le dispositif d'auto-adaptation du champ d'activités : il servira plus particulièrement à limiter le stress et la fatigue, mais aussi à adapter les activités pour limiter les expositions de la peau à des facteurs déclenchant microbiotiques et abiotiques.

Le dispositif d'auto-régulation de la conduite alimentaire : il servira à contrôler la prise d'aliments susceptibles d'aggraver la dermatite atopique, ceux qui agissent sur l'acidité de la sueur dépendant du sucre rapide et ceux qui ont une activité pro inflammatoire dépendant de la qualité des corps gras.

L'invention pour laquelle deux exemples d'application non limitatifs ont été donnés s'applique tout autant à l'optimisation des soins de la peau affectée par d'autres dermatoses inflammatoires chroniques comme les différents types d'eczéma, l'acné, le psoriasis, l'urticaire, etc., mais aussi les cancers de la peau, des dermatoses infectieuses comme l'herpès, les mycoses, le zona, etc. ou parasitaires comme la gale, etc.

Cette invention s'applique au grand organe constitué par la peau et aux muqueuses des cavités du corps et/ou aux cheveux, aux poils, à l'émail des dents, aux ongles et aux glandes sudoripares qui sont en continuité avec la peau. Ces multiples aires externes et internes présentent de grandes analogies : tout d'abord anatomo physiopathologiques, tous ayant évolué à partir de l'ectoderme comme le système nerveux. Les affections de la peau sont des marqueurs de troubles du système nerveux ou des organes des sens, mais aussi d'interaction avec les milieux microbiotiques et abiotiques individuels, et avec les activités et l'alimentation d'une personne.

Il est à noter que le système selon l'invention telle que décrite peut tout à fait être appliquée aussi bien au domaine pharmaceutique qu'à celui cosmétique.

## Revendications

1. Système de suivi dermatologique à des fins diagnostiques, thérapeutiques et/ou pronostiques, le dit système comprenant au moins :
- une première unité d'enregistrement, de stockage et d'affichage de données personnelles et cliniques de santé et d'hygiène de la peau, propres à un sujet, ladite première unité étant accessible audit sujet pour lesdits enregistrements,
- une seconde unité d'enregistrement, de stockage et d'affichage de données personnelles et cliniques de santé et d'hygiène de la peau, propres audit sujet, ladite seconde unité étant accessible à au moins un praticien médical pour lesdits enregistrements et étant connectée à ladite première unité,
- un moyen de mesure ou d'acquisition de paramètres physiques, chimiques, et/ou biologiques de l'environnement dudit sujet,
- une unité de traitement informatique connectée auxdites première et seconde unités et au moyen de mesure, ladite unité de traitement étant apte à collecter les données personnelles et cliniques des premières et secondes unités et les paramètres physiques, chimiques et/ou biologiques dudit moyen de mesure, ladite unité étant apte à alimenter une base de connaissances médicales informatisée sur la santé et l'hygiène de la peau et à être alimentée en données par ladite base,
- ladite unité de traitement informatique comprend en outre au moins un programme apte à générer, à partir desdites données personnelles et cliniques de santé et d'hygiène de la peau, desdits paramètres physiques, chimiques, et/ou biologiques, et desdites connaissances médicales informatisées :
• une consigne personnelle afin d'influer sur les états de santé et les mesures d'hygiène de la peau dudit sujet selon ce qui l'affecte; ou
• des connaissances médicales augmentées sur les soins, les états de santé et les mesures d'hygiène de la peau,
**caractérisé en ce que** les données personnelles et cliniques de santé et d'hygiène de la peau constituent un parcours de santé.

2. Système de suivi dermatologique selon la revendication **1** dans lequel ladite première unité d'enregistrement est un mobile multifonction, un ordinateur, une tablette tactile et/ou un autre appareil tactile, tel qu'une montre, borne ou cabine.

3. Système de suivi dermatologique selon la revendication **1** ou **2** dans lequel ladite seconde unité d'enregistrement est un mobile multifonction, un ordinateur, une tablette tactile et/ou un autre appareil tactile, tel qu'une montre, borne ou cabine.

4. Système de suivi dermatologique selon l'une quelconque des revendications **1** à **3** dans lequel ledit moyen de mesure ou d'acquisition de paramètres est un ou plusieurs dispositif(s) médical(aux) connecté(s) comportant des systèmes électroniques programmables et/ou à usage individuel.

5. Système de suivi dermatologique selon l'une quelconque des revendications **1** ou **4** dans lequel ledit moyen de mesure ou d'acquisition de paramètres présente des capacités d'apprentissage de type algorithme d'intelligence artificielle.

6. Système de suivi dermatologique selon l'une quelconque des revendications **1** à **5** dans lequel ledit moyen de mesure ou d'acquisition de paramètres est configuré pour acquérir :
i. des données des milieux micro-biotiques cutanés propres audit sujet ; ou
ii. des données du milieu abiotique cutané propre audit sujet ; ou
iii. des données d'activités personnelles et domestiques, professionnelles et sociales propres audit sujet ; ou
iv. des données d'alimentation propre audit sujet.

7. Système de suivi dermatologique selon l'une quelconque des revendications **1** à **6** dans lequel ladite unité de traitement informatique est connectée auxdites première et seconde unités et au moyen de mesure à travers un réseau informatique local partagé, lesdits réseaux informatiques locaux étant connectés à travers un réseau informatique global partagé comprenant un ensemble de ressources informatiques configurables pour le stockage, la transmission et la sécurité des données.

8. Système de suivi dermatologique selon l'une quelconque des revendications **1** à **7** dans lequel les transactions relatives aux traitements de données dans ladite unité de traitement informatique s'effectuent avec une technologie de type chaine de blocs dont chaque bloc regroupe les transactions propres à un ou plusieurs épisode(s) de soins de la peau.

9. Système de suivi dermatologique selon l'une quelconque des revendications **1** à **8** dans lequel ladite base de connaissances médicales informatisée est alimentée par des connaissances acquises par apprentissage du système de suivi dermatologique et des publications scientifiques.

10. Système de suivi dermatologique selon l'une quelconque des revendications **1** à **9** dans lequel lesdites première et/ou seconde unités et/ou moyen de mesure sont aptes à mettre en œuvre une ou plusieurs applications d'expérimentation clinique d'au moins un médicament et comportant au moins un protocole d'optimisation de posologie.

11. Système de suivi dermatologique selon l'une quelconque des revendications **1** à **10** dans lequel lesdites première et/ou seconde unités et/ou moyen de mesure sont aptes à mettre en œuvre une ou plusieurs applications d'expérimentation clinique d'au moins un médicament, un produit parapharmaceutique, un produit d'hygiène et/ou un produit cosmétique et de leur influence avec des médicaments.

12. Système de suivi dermatologique selon la revendication **11** dans lequel lesdites première et/ou seconde unités et/ou moyen de mesure et :
i. l'une ou plusieurs applications d'expérimentation clinique d'au moins un médicament et comportant au moins un protocole d'optimisation de posologie ou
ii. l'une ou plusieurs applications d'expérimentation clinique d'au moins un produit parapharmaceutique, un produit d'hygiène et/ou un produit cosmétique et de leur influence avec des médicaments,
sont aptes à personnaliser les indications, contre-indications et conseils sur le médicament, le produit parapharmaceutique, le produit d'hygiène et/ou le produit cosmétique, pour augmenter le service médical, de santé et/ou de beauté rendu.

13. Système de suivi médical selon l'une quelconque des revendications **1 à 12** appliqué au suivi des muqueuses tapissant les cavités du corps et/ou des cheveux, des poils, de l'émail des dents, des ongles et des glandes sudoripares, qui sont en continuité avec la peau à des fins diagnostiques, thérapeutiques et/ou pronostiques.

14. Système de suivi médical selon l'une quelconque des revendications **1 à 13** dans lequel le parcours de santé comprend une succession d'épisodes, chaque épisode comprenant au moins trois éléments :
• une observation ;
• une consigne personnelle ; et
• une évaluation de l'impact ou du résultat de cette consigne personnelle.

15. Système de suivi médical selon l'une quelconque des revendications **1 à 14** dans lequel le parcours de santé comprend un épisode d'amorçage.
